# EUROPÄISCHE PATENTANMELDUNG

(11) **EP 1 952 836 A1**
(43) Veröffentlichungstag der Anmeldung: **06.08.2008**
(21) Anmeldenummer: 07101410.4
(22) Anmeldetag: 30.01.2007
(51) Int. Cl.: A61M 5/145, A61M 5/315, A61M 5/24, A61M 5/20

(54) **Ampulle mit Verdrängungskörper**

(71) Anmelder: F.HOFFMANN-LA ROCHE AG, 4070 Basel (CH); Roche Diagnostics GmbH, 68305 Mannheim (DE)
(72) Erfinder: Heutschi, Ivan, 3506 Grosshöchstetten (CH)
(74) Vertreter: Schwabe - Sandmair - Marx

(57) **Zusammenfassung**

Die Erfindung betrifft eine Ampulle (1) zur Aufnahme einer Substanz mit einem FülIvolumen (3), in welchem die Substanz enthalten ist oder in welches die Substanz eingebracht werden kann und mit einem Verdrängungskörper (4), welcher die Substanz aus der Ampulle verdrängen kann, dadurch gekennzeichnet, dass der Verdrängungskörper so ausgebildet ist, dass das Volumen des Verdrängungskörpers variabel und insbesondere vergrößerbar ist, sowie ein System zur Abgabe einer Substanz aus einer Ampulle mit einer Ampulle nach einem der vorhergehenden Ansprüche und mit einer Saugpumpe (7), welche mit einer Ampullenöffnung oder Ampullenabgabestelle verbunden ist.

## Beschreibung

Die vorliegende Erfindung bezieht sich auf eine Ampulle, insbesondere auf eine Ampulle zur Aufnahme einer zum Beispiel medizinischen Substanz, wie zum Beispiel einer Insulin oder Hormone enthaltenden Lösung, mit einem Verdrängerkörper, wobei die in der Ampulle enthaltene Substanz mittels eines Abgabegerätes, wie zum Beispiel einer Insulinpumpe, bevorzugt genau dosiert und nach einem vorgebbaren zeitlichen Abgabeprofil, an einen Patienten abgegeben werden kann.

Eine an sich bekannte Ampulle weist einen in der Ampulle verschiebbaren Stopfen auf, wobei durch einen Druck oder eine Kraft auf den verschiebbaren Stopfen dieser in die Ampulle eingeschoben wird und so eine in der Ampulle enthaltene Substanz verdrängt, welche durch eine Abgabeöffnung aus der Ampulle abgegeben wird.

Es ist eine Aufgabe der vorliegenden Erfindung eine Ampulle zur Aufnahme einer Substanz vorzuschlagen, welche eine verbesserte oder genauere Abgabe der Substanz ermöglicht.

Diese Aufgabe wird durch eine Ampulle gemäß Anspruch 1 gelöst. Vorteilhafte Ausführungsformen ergeben sich aus den abhängigen Ansprüchen.

Eine erfindungsgemäße Ampulle zur Aufnahme einer Substanz, insbesondere einer medizinischen Substanz, wie zum Beispiel einem Medikament, Insulin, oder Hormonen, weist ein Füllvolumen auf, in welches die Substanz eingefügt wird oder in weichem die Substanz enthalten ist. Diese in dem Füllvolumen enthaltene Substanz kann über eine Abgabestelle, wie zum Beispiel eine Ampullenöffnung, die auch durch ein Septum verschlossen oder gebildet werden kann, in welches ein Abgabeelement, wie zum Beispiel ein Katheter oder eine Nadel einsticht, abgegeben werden. An oder in der Ampulle ist ein Verdrängungskörper vorhanden, welcher erfindungsgemäß so ausgestaltet ist, dass das Volumen des Verdrängungskörpers variabel oder veränveränderbar und insbesondere vergrößerbar ist, um als ein Verdrängungselement bevorzugt in der Ampulle oder in das Füllvolumen hinein mit einem sich verändernden und insbesondere sich im Ampulleninnern vergrößernden Volumen zu wirken. Dieser Verdrängungskörper kann zum Beispiel in Ergänzung zu, vor, hinter oder statt einem Stopfen in die Ampulle eingebracht werden und kann aus einem elastischen oder dehnbaren Material, wie zum Beispiel einer zusammengefalteten oder ausdehnbaren Folie oder Schicht, bestehen oder eine Hülle aus einem elastischen oder verformbaren Material aufweisen. Der Verdrängungskörper kann ein sich zum Beispiel in der Ampulle vergrößerendes Verdränger-Volumen bilden, welches mit einem Medium, zum Beispiel mit Gas, wie zum Beispiel Luft, oder mit einer Flüssigkeit, aufgefüllt wird. Dabei wird das zur Erzeugung des Verdränger-Volumens verwendete Gas oder die Flüssigkeit vorteilhaft zum Beispiel durch eine Membran oder einen Ballon von einer in der Ampulle enthaltenen Substanz abgeschirmt. Ein solcher Ballon kann sich, zum Beispiel während des allmählichen Förderns der Substanz aus der Ampulle zum Beispiel durch eine Saugpumpe, auffüllen,

Somit liegt bei einer erfindungsgemäßen Ampulle mit einem sich im Volumen verändernden und insbesondere vergrößernden Verdrängerelement vorteilhaft keine Stopfenbewegung vor, so dass es auch zu keiner Stopfenreibung an der Ampulleninnenseite kommen kann. Die bei Ampullen mit sich bewegenden Stopfen auftretenden Reibungskräften zwischen Stopfen und Ampulle können stark variieren und zum Beispiel Werte zwischen 3N und 35N annehmen. Da die erfindungsgemäße Ampulle einen Ausschütt- oder Abgabevorgang auch ohne Stopfenbewegung ermöglicht, kann die Ausschüttgenauigkeit aus der Ampulle vergrößert werden. Ein so genannter Slip-Stick oder ein Ruckgleiten eines Stopfens, was trotz mehreren Vorschüben eines Antriebs auch zu einem partiellen Nichtausschütten führen kann, wobei bei einem nachfolgenden Freigeben oder Lösen des Stopfens die Ausschüttung kumuliert erfolgt, ist bei der erfindungsgemäßen Ampulle nicht mehr möglich, wodurch die Ausschüttgenauigkeit erhöht wird.

Des Weiteren kann mit der erfindungsgemäßen Ampulle die Okklusions-Erkennung verbessert werden, da bei einer bekannten Ampulle der Schwellwert zur Detektion einer Okklusion aufgrund der möglichen Stopfenreibung relativ hoch, zum Beispiel auf 25N, eingestellt werden muss, was im Falle einer Okklusion zu einer langen Verzögerungszeit und zu einem hohen Staubolus-Volumen führt,

Da bei der erfindungsgemäßen Ampulle, welche vorgefüllt oder auch leer sein kann, kein Stopfen bewegt werden muss, um eine Substanz aus der Ampulle zu fördern, treten auch die durch die Stopfenreibung bedingten Unzulänglichkeiten nicht mehr auf.

Der erfindungsgemäße Verdrängungskörper mit variablem und vorzugsweise vergrößerbarem Volumen kann zur Erzeugung eines sich vergrößernden Verdränger-Volumens in der Ampulle verwendet werden, welches für einen Druckausgleich in der Ampulle sorgen kann. Hierdurch ist es auch möglich, dass die Ampulle von einem saugenden Element, wie zum Beispiel einer Mikro-MembranPumpe, zur Abgabe einer Substanz entleert oder ausgesogen werden kann.

Eine Ampulle mit einem solchen volumenveränderbaren Verdrängungskörper kann auf mehrere Arten realisiert werden. Beispielsweise kann der Verdrängungskörper so in der Ampulle vorgesehen sein, dass die Ampulle von einer hinteren Seite, also zum Beispiel einer Seite, welche der Abgabeöffnung oder einem Abgabebereich oder Septum der Ampulle gegenüberliegt, aufgefüllt wird, um die in der Ampulle enthaltene Substanz zu verdrängen.

Dabei ist es vorteilhaft, wenn ein zum Beispiel an einer hinteren Seite der Ampulle vorgesehener Stopfen eine Durchtrittsöffnung für das Verdränger-Medium aufweist, wobei vorzugsweise auf der Stopfeninnenseite, welche zum Beispiel an das zu fördernde Medium angrenzt, die Membran oder der sich durch das einströmende Medium ausdehnende oder entfaltende und im Ausgangszustand zusammengefaltete Ballon anliegt.

Gemäß einer weiteren Ausführungsform kann der Verdrängungskörper so angeordnet sein, dass die Verdrängung in radialer Richtung erfolgt, wobei zum Beispiel ein durch einen bevorzugt feststehenden Stopfen eingestochenes Element eine Membran oder einen Ballon trägt, der sich mit zunehmender Förderung der Substanz aus der Ampulle radial nach außen gerichtet innerhalb der Ampulle zum Beispiel von der Mittelachse der Ampulle weg ausdehnt. Beispielsweise kann eine Nadel mit einer Zuführöffnung für das Verdrängungsmedium in die Ampulle eingebracht werden oder bereits in der Ampulle vorhanden sein, welche an ihrer Umfangsseite eine oder mehrere Durchtrittsöffnungen für das Verdrängungsmedium hat Um die Nadel herum kann eine Membran oder ein Ballon angeordnet sein, welcher durch ein durch die Nadel hindurch strömendes und durch die Durchtrittsöffnung(en) austretendes Verdrängermedium allmählich während der Entnahme der Substanz aus der Ampulle aufgefüllt wird, um den Verdrängungskörper in der Ampulle sukzessive aufzufüllen und somit zu vergrößern

Bei Verwendung eines sich in radialer Richtung ausdehnenden Verdrängungskörpers kann prinzipiell auch eine bekannte Standardampulle verwendet werden, bei welcher eine Nadel mit auf der Nadelaußenseite aufliegendem Ballon oder einer auf der Nadel aufliegenden oder an der Nadel anliegenden Membran durch den Stopfen hindurch eingestochen werden kann, wobei das Verdränger-Medium durch die Nadel in den Ballon oder in die Membran gefördert werden kann, um den Verdrängerkörper volumenmäßig zu vergrößern und hierdurch für eine Verdrängung der in der Ampulle enthaltenen Substanz zu sorgen.

Gemäß einer weiteren Ausführungsform kann der Verdrängungskörper, also zum Beispiel ein Ballon oder eine sich aufblähende oder aufblasende Membran oder elastische Umhüllung, an der Innenseite der Ampulle selbst vorgesehen sein. Vorteilhaft kann der Verdrängungskörper oder die Membran zum Beispiel auch über den gesamten innenraum der Ampulle an der seitlichen und / oder hinteren oder vorderen Ampulleninnenseite umlaufend vorgesehen sein. Diese Membran oder dieser Ballon kann dann zum Beispiel durch eine seitliche Durchtrittsöffnung in der Ampulle mit dem Verdränger-Medium aufgefüllt werden.

Der Ballon oder die Membran kann aus elastischem Material bestehen, welches sich bei Auffüllung mit dem Verdränger-Medium allmählich ausdehnt. Alternativ kann der Ballon oder die Membran auch mit bereits größerer Oberfläche zum Beispiel in zusammengelegter oder zusammengefalteter Form innerhalb der Ampulle vorgesehen sein, was den Vorteil hat, dass die zur Ausdehnung der Membran erforderlich Kraft nicht mehr aufgebracht werden muss.

Bei den letzten beiden Varianten der radialen Ausdehnung des Verdrängungskörpers kann durch Geometrie der Ballone oder Dickeverhältnisse der Folie oder Membran oder durch Wahl geeigneter Materialien sichergestellt werden, dass sich der Verdrängungskörper von hinten her ausdehnt, um zu vermeiden, dass sich hinter dem Verdrängungskörper noch Materialeinschlüsse befinden, welche sich möglicherweise aufgrund eines an der Vorderseite zu weit ausgedehnten Verdrängungskörpers nicht mehr ausschütten lassen.

Als weitere Variante kann an der Vorderseite der Ampulle eine Nadel mit einem Verdrängungskörper eingestochen wird, wobei sich der Verdrängungskörper wie oben beschrieben auf der Nadelaußenseite befindet und durch ein in die Nadel einströmendes Verdrängungs-Medium aufgefüllt werden kann. Hierbei kann auch wieder eine Standard-Ampulle verwendet werden.

Besonders vorteilhaft wird die Verbindung bei Ampullen mit Luer-Anschluss verwendet, da hierbei kein Problem mit einer durch das Septum eingestochenen Nadel besteht, welche möglicherweise den Ballon beschädigen könnte.

Vorzugsweise werden der Verdränger-Körper oder die Verdränger-Elemente als Einweg-Teile oder Disposables ausgebildet und die Verdränger-Elemente können mit der Ampulle zum Beispiel über einen Schnappverschluss so gekoppelt werden, dass diese als ein Stück mit der Ampulle entsorgt werden können.

Die Erfindung wird nachfolgend anhand bevorzugter Ausführungsformen beschrieben werden.

Es zeigen:
- Figur 1: das allgemeine Funktionsprinzip der erfindungsgemäßen Ampulle;
- Figur 2: eine erste Ausführungsform der vorliegenden Ampulle mit einem hinten liegenden Verdrängungskörper;
- Figur 3: eine zweite Ausführungsform der Ampulle mit einem sich radial von innen nach außen ausdehnenden Verdrängungskörper;
- Figur 4: eine dritte Ausführungsform einer Ampulle mit einem sich radial von außen nach innen ausdehnenden Verdrängungskörper.

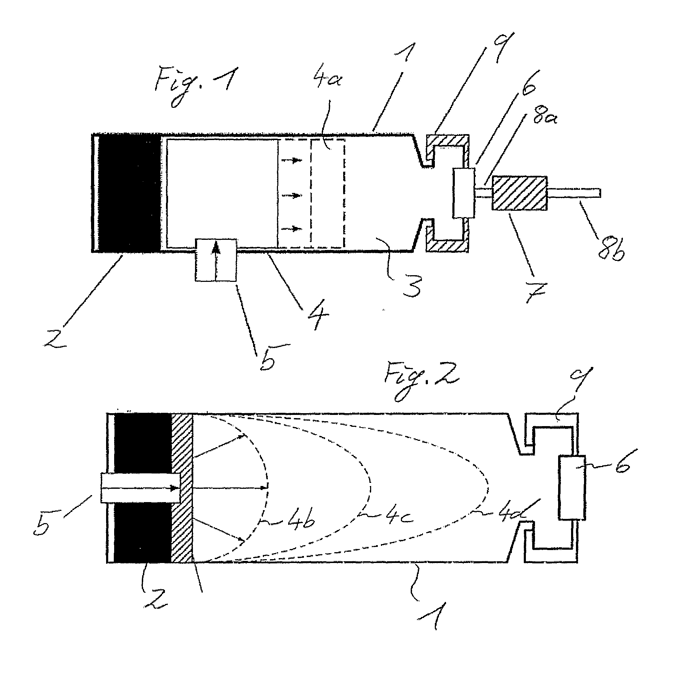

Figur 1 zeigt das allgemeine Funktionsprinzip der vorliegenden Erfindung mit einer Ampulle 1, welche an ihrem hinteren in Figur 1 links gezeigten Ende einen Stopfen 2 zur Begrenzung des Füllvolumens 3 oder zu Abdichtung der Ampulle 1 aufweist In dem in Figur 1 rechts gezeigten vorderen Teil der Ampulle 1 ist die in der Ampulle 1 enthaltene Substanz in dem Füllvolumen 3 eingebracht, welche durch den Verdrängungskörper 4, dessen Volumen sich vergrößert, wie durch den gestrichelten Verdrängungskörper 4a gezeigt, in Richtung auf die Abgabeseite der Ampulle, verdrängt werden kann. Zur Vergrößerung des Volumens des Verdränger-Körpers 4 ist an der Ampullenaußenseite ein Luft-Ventil 5 vorgesehen, über weiches zum Beispiel Umgebungsluft in den Verclrängungs-Kärper 4, 4a eingebracht werden kann, um das Verdränger-Volumen zu vergrößern. Die durch die Volumenvergrößerung des Verdrängungskörpers 4 aus der Ampulle 1 verdrängte Substanz kann zum Beispiel durch ein an der Vorderseite der Ampulle 1 in einem Ampullenverschluss 9 angeordnetes Septum 6 hindurch über eine Verbindung 8a zu einem saugenden Element, wie zum Beispiel einer Membranpumpe 7, abgegeben werden, wobei die Substanz von der Membranpumpe über eine Schlauch- oder Katheterverbindung 8b zu einem nicht gezeigten infusionsset weitergeleitet wird.

Das saugende Element oder die Membranpumpe 7 kann somit zu Beispiel Insulin aus der Ampulle 1 saugen und dieses zu dem infusions-Set fördern, wodurch Luft durch das Luft-Ventil 5 in das Verdränger-Volumen des Körpers 4, 4a gesogen wird, was für einen Druckausgleich innerhalb der Ampulle 1 ohne eine Stopfenbewegung sorgt.

Figur 2 zeigt eine erste konkrete Ausführungsform einer erfindungsgemäßen Ampulle 1 mit einem in Figur 2 gezeigten am linken hinteren Ende angeordneten verdrängenden Element oder sich ausdehnenden Ballon 4a, 4b, 4c, welcher über ein ebenfalls an der hinteren Seite der Ampulle 1 im Ampullenboden vorgesehenes und durch den Stopfen 2 hindurchgehendes Luft-Ventil 5 mit Luft befüllt werden kann. Der Stopfen 2 besteht vorzugsweise aus einem dichtenden Element, auf welchem das verdrängende Element 4 angeordnet ist und zum Beispiel aufliegt. Durch das Luft-Ventil 5 kann Luft von außen durch das dichtende Element 2 hindurch in das verdrängende Element 4 gelangen, welches zum Beispiel durch das Fördern des Insulins auf die in Figur 1 gezeigte Art immer stärker ausgedehnt wird und sich aufbläst, wie durch die gestrichelten Linien angedeutet. Da die in Figur 2 gezeigte Ampulle 1 von hinten her aufgefüllt oder ausgefüllt wird, existiert nur ein kleines Totvolumen im hinteren Teil der Ampulle 1, aus welchem eine in der Ampulle 1 enthaltene Substanz nur ungenügend oder nicht mehr abgegeben werden kann. Durch das einfache Prinzip des sich innerhalb der Ampulle 1 ausdehnenden Verdrängungskörpers 4 kann eine Ampulle 1 mit relativ wenigen Teilen einfach aufgebaut werden, was zu einer einfacheren und kostengünstigen Herstellung der Ampulle 1 führt.

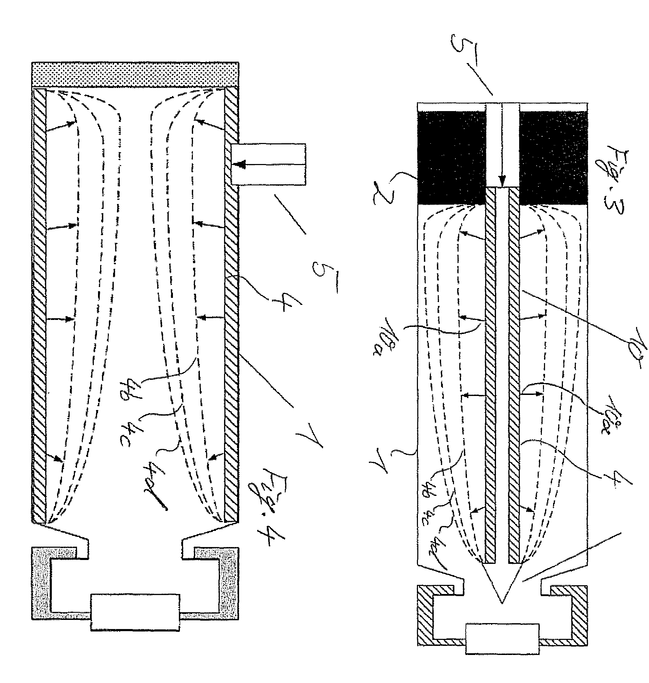

Figur 3 zeigt eine zweite konkrete Ausführungsform einer erfindungsgemäßen Ampulle 1, wobei eine Nadel 10 oder ein Füllrohr in axialer Richtung der Ampulle 1 in das Ampulleninnere eingebracht ist. Auf der Außenseite der Nadel 10 bzw des Füllrohres ist eine dehnbare Membran 4 oder ein Ballon vorgesehen, welcher sich radial nach außen innerhalb der Ampulle 1 ausdehnen kann und somit als sich vergrößernder Verdrängungskörper 4b, 4c, 4d wirken kann, wenn durch das Rohr oder die Nadel 10 hindurch, insbesondere durch einen in axialer Richtung der Nadel 10 verlaufenden Kanal hindurch, ein Verdrängungs-Medium eingeführt wird, welches zum Beispiel ebenso wie in Figur 2 gezeigt durch ein an der Ampullenhinterseite angeordnetes Luft-Ventil 5 versorgt werden kann. Die in Figur 3 gezeigte Nadel 10 mit darauf aufgebrachtem verdrängendem Element oder Ballon 4 kann auch durch den Stopfen 2 an der Ampullenhinterseite eingestochen werden

Es ist möglich, dass zum Beispiel zur Realisierung der in Figur 3 gezeigten Ausführungsform eine aus der PTCA (Percutaneous Transluminal Coronary Angioplasty) bekannte Technologie oder Prinzipien der Ballondilatation verwendet werden.

Vorzugsweise ist der sich ausdehnende Verdrängungskörper 4 oder Ballon so ausgestaltet, dass die Ampulle 1 von hinten her ausgefüllt wird, was zum Beispiel durch unterschiedliche Materialeigenschaften oder Dicken der Membran 4 oder der Verdrängungskörperoberfläche realisiert werden kann, welche sich zum Beispiel in axialer Richtung der Ampulle 1 verändern. Beispielsweise kann die Membran des Verdrängungskörpers 4 an der Ampullenhinterseite dünner sein als an der Ampullenvorderseite, so dass sich der Ballon 4, welcher beispielsweise durch mehrere in radialer und axialer Richtung der Einstechnadel 10 verteilte Öffnungen 10a aufgefüllt wird, zunächst stärker an der Ampullenhinterseite vergrößert, um zunächst die in der Ampullenhinterseite enthaltene Substanz zu verdrängen.

Die in Figur 3 gezeigte zweite Ausführungsform einer erfindungsgemäßen Ampulle 1 kann eine Verdrängung der in der Ampulle 1 enthaltenen Substanz durch eine relativ zur Ausführungsform der Figur 2 kleinere Deformation des Verdrängungskörpers 4 oder Ballons realisieren.

Figur 4 zeigt eine dritte Ausführungsform einer erfindungsgemäßen Ampulle 1 mit einen am Ampullenrand und bevorzugt umlaufend an der Ampulleninnenseite vorgesehenen Verdrängungskörper 4 oder Membran, welche von einem zum Beispiel seitlich in der Ampullenwand vorgesehenen Luftventil aufgefüllt werden können Bei der Förderung der in der Ampulle 1 enthaltenen Substanz kann die auf der Innenseite der Ampulle 1 vorgesehene Membran-Schicht 4 oder Ballon-Schicht mit Luft gefüllt werden und hierdurch für einen Druckausgleich sorgen

Dabei kann der Verdränger-Körper oder die Membran-Schicht 4 oder Ballon-Schicht auf der gesamten Ampulleninnenseite angebracht oder vorgesehen sein oder auch nur an einem Teilstück der Ampulle 1, wie zum Beispiel nur über die halbe Umfangsseite der Ampulle.

## Patentansprüche

1. Ampulle zur Aufnahme einer Substanz mit einem Füllvolumen (3), in welchem die Substanz enthalten ist oder in welches die Substanz eingebracht werden kann und mit einem Verdrängungskörper (4), welcher die Substanz aus der Ampulle (1) verdrängen kann,
**dadurch gekennzeichnet, dass**
der Verdrängungskörper (4) so ausgebildet ist, dass das Volumen des Verdrängungskörpers (4) variabel und insbesondere vergrößerbar ist.

2. Ampulle nach Anspruch 1 mit einer Abgabestelle oder Öffnung oder einem Septum (6), durch welches eine in der Ampulle (1) enthaltene Substanz abgegeben werden kann.

3. Ampulle nach einem der vorhergehenden Ansprüche, wobei der mit einem Medium befüllbare Verdrängungskörper (4) durch eine Membran, ein elastisches oder dehnbares Material, oder eine elastische Folie begrenzt wird.

4. Ampulle nach einem der vorhergehenden Ansprüche, wobei der Verdrängungskörper (4) eine zusammengefaltete Folie oder Membran ist.

5. Ampulle nach dem vorhergehenden Anspruch, wobei das verformbare Material (4) an der Ampullenhinterseite vorgesehen ist.

6. Ampulle nach einem der vorhergehenden Ansprüche, wobei das elastische Material (4) an der Ampulleninnenseite oder Ampullenseitenwand vorgesehen ist.

7. Ampulle nach einem der vorhergehenden Ansprüche mit einer Verdrängungsmedium-Zuführvorrichtung oder Nadel (10), welche bevorzugt in axialer Richtung der Ampulle (1) vorgesehen ist und an welcher ein Verdrängungskörper oder eine Membran (4) vorgesehen ist.

8. Ampulle nach dem vorhergehenden Anspruch, wobei der Verdrängungskörper oder die elastische Membran (4) umlaufend um die Zuführvorrichtung oder Nadel (10) vorgesehen ist.

9. Ampulle nach einem der vorhergehenden Ansprüche mit einem Einlassventil (5) für ein Verdrängungsmedium, welches mit einer Seite des Verdrängungskörpers (4) oder einer elastischen Membran verbunden ist, welche der Seite der Membran gegenüberliegt, welche zu der in der Ampulle (1) enthaltenen Substanz weist oder an die Substanz angrenzt.

10. Ampulle nach einem der vorhergehenden Ansprüche, wobei der Verdrängungskörper (4) mit der Ampulle (1) fest verbunden oder über einen Schnappverschluss gekoppelt ist.

11. System zur Abgabe einer Substanz aus einer Ampulle (1) mit einer Ampulle (1) nach einem der vorhergehenden Ansprüche und mit einer Saugpumpe (7), welche mit einer Ampullenöffnung oder Ampullenabgabestelle (6) verbunden ist.

12. System nach dem vorhergehenden Anspruch, wobei die Saugpumpe (7) eine Membranpumpe oder eine Silizium-Mikropumpe ist.
